# EUROPEAN PATENT APPLICATION

(11) **EP 1 288 665 A1**
(43) Date of publication of application: **05.03.2003**
(21) Application number: 01930236.3
(22) Date of filing: 21.05.2001
(51) Int. Cl.: G01N 33/564

(54) **METHOD OF ASSAYING ANTI-ENA ANTIBODY AND ASSAY KIT**

(30) Priority: 26.05.2000 JP 2000157410
(71) Applicant: Medical & Biological Laboratories Co., Ltd., Nagayo-shi, Aichi 460-0002 (JP)
(72) Inventor: MURAKAMI, Akihiro, Ina-shi, Nagano 396-0111 (JP); KOJIMA, Kazuo, Ina-shi, Nagano 396-0113 (JP)
(74) Representative: Knauthe
(86) International application number: JP0104251
(87) International publication number: WO01090756

(57) **Abstract**

A method for measuring an anti-ENA antibody having a high correlation with the conventional DID method and having a high sensitivity is provided. A complex of an RNA which is substantially the same as an RNA constituting ENA (Extractible Nuclear Antigen) and a protein molecule which is substantially the same as a intracellular non-histone soluble protein constituting ENA is formed and the relevant complex and a specimen are reacted. The resultant reaction product is detected.

SEQUENCE LISTING

<110> MEDICAL & BIOLOGICAL LABORATORIES CO., LTD.
Murakami, Akihiro
Kojima, Kazuo

<120> Methods and Kits for measuring antibodies against ENA

<130> P021101

<140>
<141>

<150> JP P2000-157410
<151> 2000-05-26

<160> 12

<170> PatentIn Ver. 2.1

<210> 1
<211> 31
<212> DNA
<213> Artificial Sequence

<220>
<223> Description of Artificial Sequence:Artificially
synthesized primer sequence

<400> 1
caggggaaag cgcgaacgca gtcccccact a 31

<210> 2
<211> 32
<212> DNA
<213> Artificial Sequence

<220>
<223> Description of Artificial Sequence:Artificially
synthesized primer sequence

<400> 2
atacttacct ggcaggggag ataccatgat ca 32

<210> 3
<211> 164
<212> DNA
<213> Artificial Sequence

<220>
<223> Description of Artificial Sequence:Artificially
      synthesized primer sequence

<400> 3
atacttacct ggcaggggag ataccatgat cacgaaggtg gttttcccag ggcgaggctt 60
atccattgca ctccggatgt gctgacccct gcgatttccc caaatgtggg aaactcgact 120
gcataatttg tggtagtggg ggactgcgtt cgcgctttcc cctg 164

<210> 4
<211> 30
<212> DNA
<213> Artificial Sequence

<220>
<223> Description of Artificial Sequence:Artificially
      synthesized primer sequence

<400> 4
atgacccagt tcctgccgcc caaccttctg 30

<210> 5
<211> 30
<212> DNA
<213> Artificial Sequence

<220>
<223> Description of Artificial Sequence:Artificially
      synthesized primer sequence

<400> 5
ctccggcgca gcctccatca aatacccatt 30

<210> 6
<211> 1314
<212> DNA

<213> Artificial Sequence

<220>
<223> Description of Artificial Sequence:Artificially
    synthesized primer sequence

<400> 6
atgacccagt tcctgccgcc caaccttctg gccctctttg ccccccgtga ccctattcca 60
tacctgccac ccctggagaa actgccacat gaaaaacacc acaatcaacc ttattgtggc 120
attgcgccgt acattcgaga gtttgaggac cctcgagatg cccctcctcc aactcgtgct 180
gaaacccgag aggagcgcat ggagaggaaa agacgggaaa agattgagcg gcgacagcaa 240
gaagtggaga cagagcttaa aatgtgggac cctcacaatg atcccaatgc tcagggggat 300
gccttcaaga ctctcttcgt ggcgagagtg aattatgaca caacagaatc caagctccgg 360
agagagtttg aggtgtacgg acctatcaaa agaatacaca tggtctacag taagcggtca 420
ggaaagcccc gtggctatgc cttcatcgag tacgaacacg agcgagacat gcactccgct 480
tacaaacacg cagatggcaa gaagattgat ggcaggaggg tccttgtgga cgtggagagg 540
ggccgaaccg tgaagggctg gaggccccgg cggctaggag gaggcctcgg tggtaccaga 600
agaggagggg ctgatgtgaa catccggcat tcaggccgcg atgacacctc ccgctacgat 660
gagaggcccg gcccctcccc gcttccgcac agggaccggg accgggaccg tgagcgggag 720
cgcagagagc ggagccggga gcgagacaag gagcgagaac ggcgacgctc ccgctcccgg 780
gaccggcgga ggcgctcacg gagtcgcgac aaggaggagc ggaggcgctc cagggagcgg 840
agcaaggaca aggaccggga ccggaagcgg cgaagcagcc ggagtcggga gcgggcccgg 900
cgggagcggg agcgcaagga ggagctgcgt ggcggcggtg gcgacatggc ggagccctcc 960
gaggcgggtg acgcgccccc tgatgatggg cctccagggg agctcgggcc tgacggccct 1020
gacggtccag aggaaaaggg ccgggatcgt gaccgggagc gacggcggag ccaccggagc 1080
gagcgcgagc ggcgccggga ccgggatcgt gaccgtgacc gtgaccgcga gcacaaacgg 1140
ggggagcggg gcagtgagcg gggcagggat gaggcccgag gtgggggcgg tggccaggac 1200
aacgggctgg agggtctggg caacgacagc cgagacatgt acatggagtc tgagggcggc 1260
gacggctacc tggctccgga gaatgggtat ttgatggagg ctgcgccgga gtga 1314


<210> 7
<211> 30
<212> DNA
<213> Artificial Sequence

<220>
<223> Description of Artificial Sequence:Artificially
    synthesized primer sequence

<400> 7
atggcagttc ccgagacccg ccctaaccac 30


<210> 8

<211> 29
<212> DNA
<213> Artificial Sequence

<220>
<223> Description of Artificial Sequence:Artificially
      synthesized primer sequence

<400> 8
cttcttggca aaggagatct tcatggcgt                                   29


<210> 9
<211> 849
<212> DNA
<213> Artificial Sequence

<220>
<223> Description of Artificial Sequence:Artificially
      synthesized primer sequence

<400> 9
atggcagttc ccgagacccg ccctaaccac actatttata tcaacaacct caatgagaag 60
atcaagaagg atgagctaaa aaagtccctg tacgccatct tctcccagtt tggccagatc 120
ctggatatcc tggtatcacg gagcctgaag atgaggggcc aggcctttgt catcttcaag 180
gaggtcagca gcgccaccaa cgccctgcgc tccatgcagg gtttcccttt ctatgacaaa 240
cctatgcgta tccagtatgc caagaccgac tcagatatca ttgccaagat gaaaggcacc 300
ttcgtggagc gggaccgcaa gcgggagaag aggaagccca agagccagga gaccccggcc 360
accaagaagg ctgtgcaagg cgggggagcc acccccgtgg tgggggctgt ccaggggcct 420
gtcccgggca tgccgccgat gactcaggcg ccccgcatta tgcaccacat gccgggccag 480
ccgccctaca tgccgccccc tggtatgatc cccccgccag gccttgcacc tggccagatc 540
ccaccagggg ccatgccccc gcagcagctt atgccaggac agatgccccc tgcccagcct 600
ctttctgaga atccaccgaa tcacatcttg ttcctcacca acctgccaga ggagaccaac 660
gagctcatgc tgtccatgct tttcaatcag ttccctggct tcaaggaggt ccgtctggta 720
cccgggcggc atgacatcgc cttcgtggag tttgacaatg aggtacaggc aggggcagct 780
cgcgatgccc tgcagggctt taagatcacg cagaacaacg ccatgaagat ctcctttgcc 840
aagaagtag                                                         849


<210> 10
<211> 480
<212> DNA
<213> Artificial Sequence

<220>

<223> Description of Artificial Sequence:Artificially
synthesized primer sequence

<400> 10
atgcccaagt tttattgtga ctactgcgat acatacctca cccatgactc tccatctgtg 60
agaaagacac actgcagtgg aaggaaacac aaagagaatg tgaaagacta ttatcagaaa 120
tggatggaag agcaggctca gagcctgatt gacaaaacaa cggctgcatt tcaacaagga 180
aagatacctc ctactccatt ctctgctcct cctcctgcag gggcgatgat accacctccc 240
cccagccttc cgggtcctcc tcgccctggt atgatgccag caccccatat ggggggccct 300
cccatgatgc caatgatggg ccctcctcct cctgggatga tgccagtggg acctgctcct 360
ggaatgaggc cgcccatggg aggccatatg ccaatgatgc ctgggccccc aatgatgaga 420
cctcctgccc gtcccatgat ggtgcccact cggcccggaa tgactcgacc agacagataa 480

<210> 11
<211> 30
<212> DNA
<213> Artificial Sequence

<220>
<223> Description of Artificial Sequence:Artificially
synthesized primer sequence

<400> 11
atgcccaagt tttattgtga ctactgcgat 30

<210> 12
<211> 30
<212> DNA
<213> Artificial Sequence

<220>
<223> Description of Artificial Sequence:Artificially
synthesized primer sequence

<400> 12
tctgtctggt cgagtcattc cgggccgagt 30

## Description

### FIELD OF THE INVENTION

The present invention relates to a method and a kit for measuring an anti-ENA antibody. More particularly, the present invention relates to a method and a kit for measuring an anti-ENA antibody using ENA (Extractable Nuclear Antigen) which is an intracellular non-histone soluble protein and an RNA complex to which an ENA binds.

### BACKGROUND OF THE INVENTION

Collagen disease is a disease concept that has been proposed by P. Klempererin 1942, and a general name of the diseases in which fibrinoid degeneration is commonly recognized in systemic connective tissues. Although initially, the concept of the collagen disease was defined to include six diseases such as systemic erythematodes, scleroderma and the like, at present, the concept of collagen disease seems to have a broader meaning. Immune abnormality is one of the common pathogens or clinical features in collagen disease. Among the features, the production of autoantibodies is characteristic, and it is known that in the serum of a patient with collagen disease, there appear a variety of autoantibodies. As for these autoantibodies, many researchers have studied and elucidated their relationship with respective diseases and clinical features, the detection and measurement of the specific autoantibody which is considered to be available for the concrete identification of the disease or diagnosis, pursuit of the pathogen, the determination of therapeutic effect and the estimation of the prognosis.

Among autoantibodies appearing in the serum of a patient with collagen disease, an antibody group against extractable nuclear antigen (ENA) extracted from the cell nucleus with an isotonic buffer is called an anti-ENA antibody. As an anti-ENA antibody, a variety of antibodies which are called from their corresponding antigens as anti-RNP (ribonucleoprotein) antibody, anti-Sm antibody, anti-SS-A antibody, anti-SS-B antibody and the like are known. Conventionally, the measurement of the anti-ENA antibody has been performed by a double immunodiffusion method (DID method).

Although the DID method is high in specificity, it is difficult to measure a large number of specimens at the same time, and moreover, the fact that it is difficult to objectively evaluate the measured results is considered to be a problem. On the other hand, as a method for measuring a specimen which substitutes for the DID method, an ELISA (enzyme-linked imunosorbent assay) method has been proposed using an antigen corresponding to the respective anti-ENA antibodies. Although the ELISA method has an advantage such that it easily and objectively determines the results in addition to an advantage such that a large number of specimens can be treated by a simple and easy operation at the same time, however, among the specimens determined to be positive in the DID method, there exist some specimens determined to be negative by the ELISA method, the dissociation phenomenon has been considered to be a problem (Hiroshi, Sakai et al., Medicine and Pharmacology 21(5): 957-60,1989). Hereinafter, an anti-RNP antibody which is one species of anti-ENA antibodies will be exemplified and the concrete problems will be described.

An anti-U1RNP antibody is an autoantibody whose frequent emergences are recognized in the serum of a patient with autoimmune disease such as SLE (systemic erythematodes), MCTD (mixed connective tissue disease) or the like. It is said that the corresponding antigens of this anti-U1RNP antibody are RNP (ribonucleoprotein) 70k protein (70 kDa), RNPA protein (33 kDa) and RNPC protein (22 kDa)(hereinafter, these are in general referred to as "RNP protein") (Tan, E.M., Adv. Immunol. 44: 93-152, 1989). It is known that these RNP proteins exist as a complex bound to small nuclear RNAs which are referred to as U1snRNA (165 bases) in vivo (Venrooij, W. J., J. Rheumatol. 14: 78-82, 1987). It is also known that the proteins which are referred to as SmB', SmB, SmD, SmE, SmF and SmG in addition to RNP protein also bind to the U1snRNA. thereby form a large ribonucleic acid-protein complex and exist in vivo.

In the case where an anti-U1RNP antibody is measured by a DID method, a so-called crude antigen (DID antigen) which has been extracted from a pulverized mammal animal cell is used. In the DID method, first, a DID antigen and a specimen are put into separate spots provided in a gel layer such as agar or the like, and both are diffused within the gel layer. In the case where an anti-ENA antibody is contained in a specimen, the precipitates of the anti-ENA antibody and the DID antigen are formed within the gel layer, and the precipitates are observed as precipitation lines. The determination of whether the specimen is positive or negative as to anti-U1RNA antibody is made by whether these precipitation lines and a control precipitation line formed by using the already known anti-RNP antibody positive specimen are fused or not.

On the other hand, in the case where an anti-U1RNP antibody is measured by an ELISA method, the above-described three species of proteins (RNP70k, RNPA and RNPC) which are constitution factors of U1RNP are prepared, and one, which is solid-phased, has been used in general. This is because these proteins have been considered antigens of the anti-U1RNP antibody. However, the fact that an anti-U1RNP antibody, which recognizes not these protein portions but U1sn exists, has been confirmed by Wilusz and Keene et al.(Wilusz, J.,Keene, J.D., J. Biol. Chem. 261 (12): 5497-72, 1986). Therefore, it is estimated that the fact that the anti-U1RNP antibody, which recognizes not this protein portion but nucleic acid (U1snRNA portion, could not have been detected is one factor for the dissociation phenomenon in the determination results by the DID method and by the ELISA method. Moreover, it is also estimated that RNA protein complex indicates a new antigen recognition site by sterically forming an RNA protein complex.

The problem to be solved by the present invention is to provide a novel method for measuring an anti-ENA antibody, which overcomes the above-described drawbacks in the conventional method for measuring an anti-ENA antibody.

### SUMMARY OF THE INVENTION

In order to solve the above-described problems, first, the present inventors have examined the DID method by using an anti-U1RNP antibody as follows.

In the case where the anti-U1RNP antibody positive specimens have been measured using a crude antigen (hereinafter, referred to as "DID antigen") used in the DID method, one precipitation line is formed. The determination of whether each specimen is positive or negative is performed by whether this precipitation line and the precipitation line, which is formed by using the known anti-RNP antibody positive specimen used as a control, are fused with each other or not (Hiroshi, Sakai et al., Medicine and Pharmacology 21 (5): 957-60, 1989). When a similar measurement has been performed using this ribonuclease treated crude antigen, the phenomenon that the precipitation line disappeared in a positive specimen was frequently observed. From this fact, it has been expected that an RNP protein in the DID antigen exists in a state of forming a complex with Because, if three species of RNP proteins whose molecular weights are different individually exist in the DID antigen, as for the precipitation line obtained by reacting with the anti-U1RNP antigen positive specimen, it is considered that three precipitation lines should be possibly observed at a maximum. But depending upon the specimen, the disappearance of the precipitation lines may not possibly occur in theory even if the DID antigen is ribonuclease-treated.

Moreover, the reactivity between an anti-U1RNP antibody and an U1snRNA in the specimen has been examined by an immune precipitation method and an ELISA method (see (3) of (Reference Experiment 1] described later). As described above, since the existence of the anti-U1RNP antibody recognizing not RNP protein portion but U1snRNA has been proved, it might be considered that the dissociation specimens, which are determined as negative by an ELISA method using an RNP protein but which are determined as positive by a DID method, have the reactivity with U1snRNA. Hence, U1snRNA was prepared by in vitro transcription method (see [Example 1] described later), the measurement of the dissociation specimens has been performed using this U1snRNA by an immune precipitation method and ELISA method. As a result of this, it is understood that in both measurement methods, a portion of specimens has reactivity with U1snRNA

Subsequently, U1snRNA and three species of RNP proteins were individually prepared, these were mixed in PBS buffer, and in an ELISA system which has been solid-phased, there were results that all of the dissociation specimens which had been used were determined to be positive. This fact strongly suggests that an U1snRNA formed a complex with RNP proteins in the buffer and this complex was solid-phased. Moreover, the results suggest that the anti-U1RNP antigen recognizing the U1snRNA-RNP protein complex exists in addition to the anti-U1RNP antigen which individually recognizes the U1snRNA and the three species of RNP proteins among the dissociation specimens.

The present invention is based on the above-described considerations and the acknowledgement obtained thereby, the first aspect of the present invention comprises the following constitution:

A method for measuring an anti-ENA antigen which specifically recognizes an antigen containing a first RNA and a first intracellular non-histone soluble protein, the method having the step in which a complex of a second RNA molecule which is substantially the same as the first RNA and the first protein molecule which is substantially the same as the first intracellular non-histone soluble protein is formed, then the complex and the specimen are reacted.

According to the method for measuring an anti-ENA antibody of the above-described constitution, first, a complex is formed using an RNA and a protein which are substantially the same as an RNA and an intracellular non-histone soluble protein in the antigen of anti-ENA antibody, respectively. Specifically, an antigen (complex) nearly in the state in vivo is reconstructed, and the reactivity (connectivity) between this and an anti-ENA antibody in the specimen is measured. Therefore, an anti-ENA antibody capable of recognizing an antigen in the state of being in vivo can be detected with good sensitivity. This also means that the measurement result difference occurring between the case where conventionally it is measured using a crude antigen extracted from the organism material and the case where it is measured by making only a intracellular non-histone soluble protein as an antigen can be solved. Specifically, when a DID method (Double Immune Diffusion method) is exemplified, an antigen used therein is an extracted liquid obtained by making a mammal tissue as a starting material, and in such an extracted liquid, it is considered that almost all of the antigen (ENA) do not exist independently, but exist in the state where they bound to an inherent RNA. Therefore, in the conventional DID method, the amount of the anti-ENA antibody which recognizes ENA in the state where it is bound to RNA has been measured. On the other hand, a measurement of an anti-ENA antibody by an ELISA method in which an intracellular non-histone soluble protein that the anti-ENA antibody of the measuring object recognizes is prepared and this is made as an antigen has been also proposed. However, in this case, an anti-ENA antibody which recognizes an intracellular non-histone soluble protein independently existing is to be measured. In this way, since in the DID method and ELISA method, the measurements are performed using different antigens, the cases where the matching or correlation between the measurement results is not observed have occurred. In a constitution in the first aspect of the present invention, as described above, since the measurement of an anti-ENA antibody is performed using an antigen in a state where it is nearly in an organism, the measurement having a high correlation with the conventional methods such as a DID method using an extracted liquid from an organism material or the like can be performed.

Moreover, since a complex (antigen) used in the first aspect of the present invention can be easily prepared using a genetic engineering technique, a biochemical technique or the like, the simplification of the measurement operations is contemplated, and the product of a stable quality is capable of being supplied.

Furthermore, since in the first aspect of the present invention, a newly reconstructed complex is used as an antigen, the influence of the contaminants in the antigen (solution) can be more largely reduced and the enhancement of the measurement sensitivity can be more highly expected than the case where the extracted matter from an organism tissue was made as an antigen solution.

In addition, from the viewpoint of the comparison with the DID method, a large number of antigens with very little inclusion of impurities can be prepared, it also has an advantage that a large number of specimens can be measured in a short time since the constitution of the present invention is capable of being applied to an ELISA method or the like which is capable of measuring a large number of specimens at the same time.

### BRIEF DESCRIPTION OF THE DRAWINGS

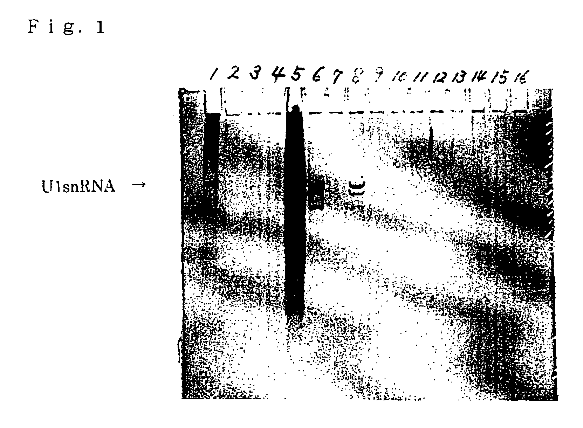

Fig. 1 is a figure showing a gel dyed after the samples obtained as a result of performing the immune precipitation method on the dissociation specimens of 14 specimens (serum 1-serum 14) were electrophoresis in (3) of the Reference Experiment 1. The samples prepared from the dissociation specimens were separately flowed in lanes 3-16. As a control, in lane 1, an U1snRNA (positive control) prepared by an in vitro transcription method of Example 1 and in lane 2, the sample prepared from serum of the healthy person (negative control) were separately flowed. The arrow shows the band location of the U1snRNA.

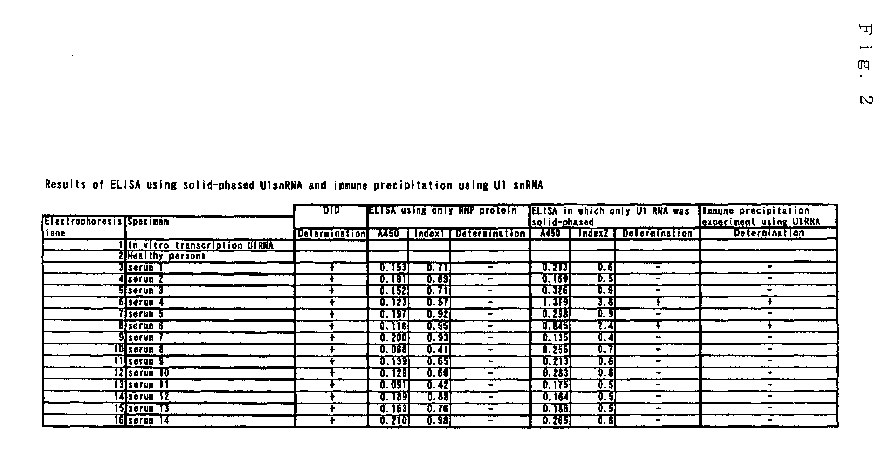

Fig. 2 is a table in which the results by the immune precipitation method and the ELISA method in (3) of the Reference Experiment Example 1 are compiled. It should be noted that the measurement results of the respective dissociation specimens by the DID method in (1) of the same Experiment Example and by the conventional type ELISA method (ELISA method that a plate on which only RNP protein is solid-phased is used) in (2) of the same Experiment Example are also indicated.

As for Index 1, from the fact that on the ELISA plate where only RNP protein was solid-phased, the average value + 3 x the standard deviation of A450 value that 100 examples of the healthy persons' specimens were measured was 0.215, and A450/0.215 of the respective serums were calculated. In the determination 1, it was defined that in the case where the value of Index 1 is one or more, it was determined as being positive (+), and in the case where the value of Index 1 is one or less, it was determined as negative (-).

As for Index 2, from the fact that on the ELISA plate where only RNP protein was solid-phased, the average value + 3-x the standard deviation of A450 value that 100 examples of the healthy persons' specimens were measured as 0.350, and A450/0.350 of the respective serums were calculated. In the determination 2, it was defined that in the case where the value of Index 2 is one or more, it was determined as being positive (+), and in the case where the value of Index 1 is one or less, it was determined as negative (-).

In the determination 3, in the case where the band of U1snRNA was observed in the gel shown in Fig. 1, it is determined as being positive (+), and in the case where the band of U1snRNA was not observed in the gel shown in Fig. 1, it is defined as negative (-).

The serums 1-14 belong to a dissociation specimen group, which were determined as positive (+) in the measurement by the DID method, and which were determined as negative (-) in the measurement by the ELISA method in which only RNP protein was solid-phased.

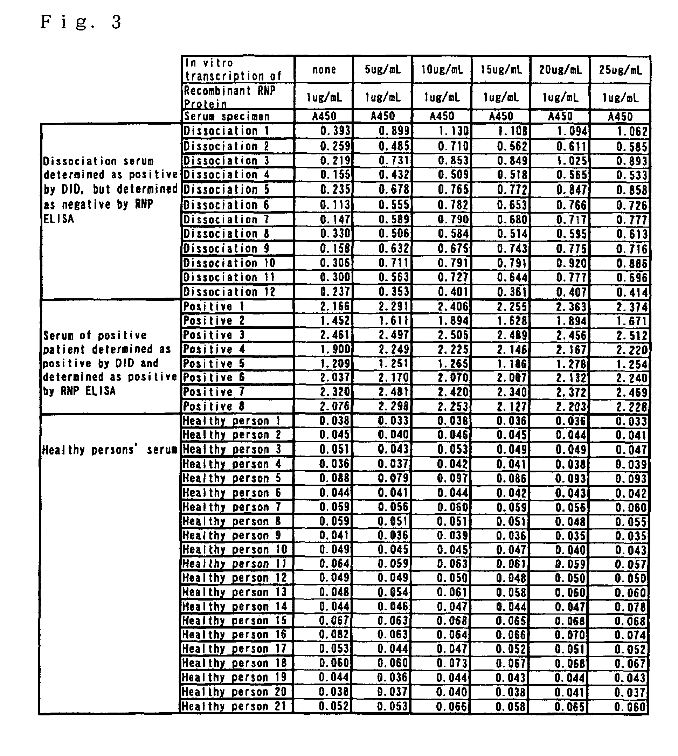

Fig. 3 is a table in which the measurement results by the ELISA method in Example 5 are compiled. The dissociation specimens are represented as the dissociation 1-dissociation 12. In a similar manner, the positive specimens are individually represented as the positive 1-positive 8, the healthy persons' specimens are individually represented as the healthy person 1-healthy person 21.

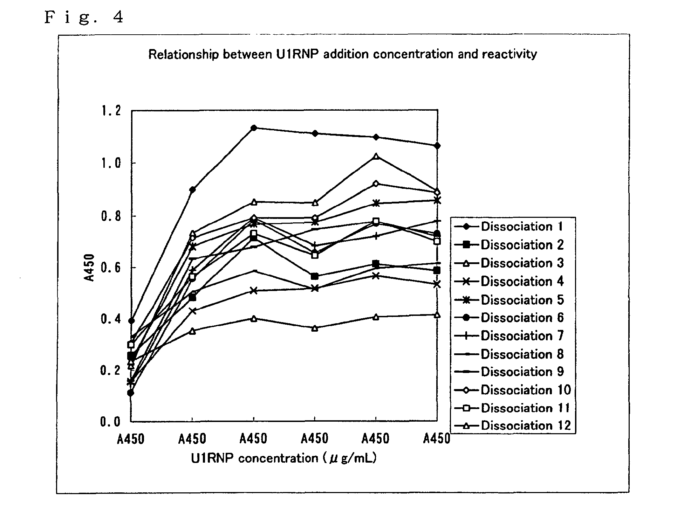

Fig. 4 is a graph plotting the measurement results (dissociation specimens 1-12) of the ELISA method in Example 5.

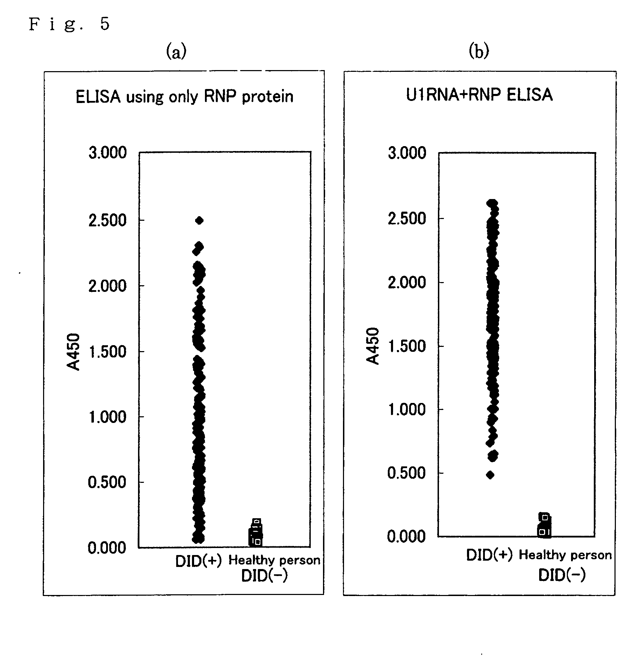

Fig. 5 is a graph showing the measurement results of the ELISA method in Example 6. Fig. 5 (a) is a graphical representation plotting the measurement results using an RNP-ELISA plate, Fig. 5 (b) is a graphical representation plotting the measurement results using U1snRNA + RNP ELISA plate. In both graphical representations, the measurement results of the specimen group of being positive by the DID method are located on the left side, and the measurement results of the specimen group of being negative by the DID method are located on the right side.

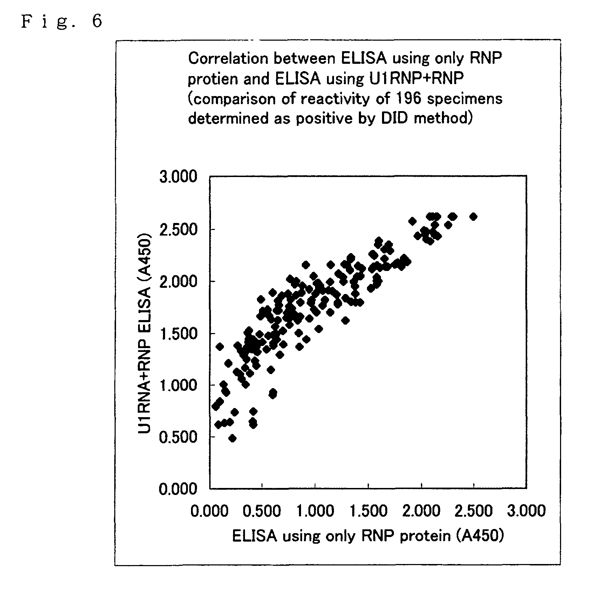

Fig. 6 is a graph showing the measurement results of Example 6. The correlation between the conventional ELISA method (system in which only RNP protein was used as a solid-phased antigen) and an ELISA method according to the present invention (system in which the complex made of U1snRNA and RNP was used as a solid-phased antigen) is shown. Only the measurement results of the 196 serum specimens of the patients with collagen disease who were determined as being positive by the DID method were plotted. The abscissa axis shows a color development (A450) by the ELISA method in which only RNP protein was used as a solid-phased antigen, and the ordinate axis shows a color development (A450) in which U1snRNA and RNP protein were used as solid-phased antigens.

### DESCRIPTION OF THE PREFERRED EMBODIMENTS

An "antigen" in the first aspect of the present invention is constituted by containing the first RNA (ribonucleic acid) and the first intracellular non-histone soluble protein. Specifically, in the first aspect of the present invention, an anti-ENA antibody which specifically recognizes the antigen constituted by containing an RNA and an intracellular non-histone soluble protein in vivo is measured. Moreover, it also includes one constituted by containing the second intracellular non-histone soluble protein in addition to the first RNA and the first intracellular non-histone soluble protein as an antigen.

The first RNA is an RNA to which the first intracellular non-histone soluble protein described later specifically binds, and is inherent in this intracellular non-histone soluble protein. For example, these are U1snRNA U2snRNA, hY1-5snRNA, rRNA and the like. The first intracellular non-histone soluble protein contains the known ENA (Extractable Nuclear Antigen), for example, which is any one of U1RNP, Sm, SS-A, SS-B, Jo-1 and PM-Scl. Moreover, needless to say, there are cases where the first intracellular non-histone soluble protein comprises a single protein, and there are also the cases where it comprises an assembly of proteins. For example, in the case of RNPs, the first intracellular non-histone soluble proteins can be made with RNP-70k protein (70 kDa), RNP-A protein (33 kDa) and RNP-C protein (22 kDa). Needless to say, the first intracellular non-histone soluble proteins can be also made with one or two species of proteins optionally selected from these 3 species of proteins.

In the case where an antigen contains the second intracellular non-histone soluble protein, the relevant second intracellular non-histone soluble protein is a protein molecule; which is different from the first intracellular non-histone soluble protein, and which binds to the first RNA at a site different from the site to which the first intracellular non-histone soluble protein binds. As a second intracellular non-histone soluble protein, for example, U1RNP, Sm, SS-A, SS-B, Jo-1 or PM-Scl can be listed. Moreover, similar to the first intracellular non-histone soluble protein, needless to say, there are the cases where it comprises a single protein, and there are also the cases where it comprises an assembly of proteins. For example, the second intracellular non-histone soluble protein can be made of Sm proteins comprising an assembly of SmB', SmB, SmE, SmF and SmG.

In the present specification, the term "anti-ENA antibQdy" is an antibody which specifically recognizes the antigen containing the first RNA and its corresponding first intracellular non-histone soluble protein. Therefore, anti-ENA antibodies include an antibody which specifically recognizes an intracellular non-histone soluble protein portion , an antibody which recognizes an RNA portion to which the relevant intracellular non-histone soluble protein binds and an antibody which recognizes a complex portion of the relevant intracellular non-histone soluble protein and the relevant RNA. The portion formed by the specific conformation followed by the formation of the complex of an intracellular non-histone soluble protein and an RNA is one of the examples of the complex portion. For example, in the case where the first RNA is U1sn and the first intracellular non-histone soluble protein is an RNP, the anti-U1RNP antibody is relevant to an anti-ENA antibody. Moreover, in the case where the first RNA is U1snRNA the first intracellular non-histone soluble protein is made Sm, an anti-Sm antibody is relevant to an anti-ENA antibody. Here, in the case where the antigen contains the second intracellular non-histone soluble protein, the anti-ENA antibody is an assembly of antibodies which recognize the first RNA, the first intracellular non-histone soluble protein or the complex of these two components, but it does not contain an antibody which recognizes the second intracellular non-histone soluble protein portion.

In a method for measurement of the first aspect of the present invention, first, a complex of the second RNA which is substantially the same as the first RNA contained in the antigen and the first protein molecule which is substantially the same as the above-described first intracellular non-histone soluble protein(hereinafter, referred to as "RNA protein complex") is formed. Even in the case where the antigen contains the second intracellular non-histone soluble protein, a complex of the second RNA which is substantially the same as the first RNA and the first protein molecule which is substantially the same as the above-described first intracellular non-histone soluble protein is formed.

Here, the phrase "substantially the same as" is referred to a molecule which can form the functional conformation in the connectivity with an anti-ENA antibody, thereby the second RNA can be, for example, an RNA whose base sequence is the same as that of the first RNA or an RNA whose base sequence is similar but different from the base sequence of the first RNA by partial deletion, partial substitution, or partial addition. Preferably, an RNA having a base sequence which is the same as the first RNA is used. It should be noted that it is required that the second RNA could at least bind to the first protein molecule. Ones being substantially the same in the first protein molecule include, for example, a protein whose amino acid sequence is the same as that of the first intracellular non-histone soluble protein, and a protein whose amino acid sequence is similar but different from the amino acid sequence of the first intracellular non-histone soluble protein by partial deletion, partial substitution, or partial addition. Preferably, a protein having the same amino acid sequence as that of the first intracellular non-histone soluble protein is used. As described above, in the case where the first intracellular non-histone soluble protein is comprised of the assembly of two or more proteins, it is preferable that the assembly of the protein molecules which is substantially the same as the relevant protein in each of assembly of the of the above two or more proteins is used as the first protein molecule. It should be noted that the first protein molecule is at least required to be capable of binding to the second RNA.

Such a second RNA can be prepared by a chemical synthesis, for example, on the basis of the base sequence of the known first RNA. Moreover, the above-described first RNA is purified from a mammal tissue or a mammal cultured cell using the known biochemical techniques and the genetic engineering techniques, which can be also used as the second RNA. Moreover, on the basis of the purified first RNA, it is amplified by the known gene engineering procedure, and then, it can be made as the second RNA. For example, an anti-ENA antibody in the human serum which is anti-ENA antibody positive is bound to the carrier such as protein-A-Sepharose or the like, to which a suitable cell extract (for example, HeLa cell extract) or the like is added, thereby trapping the antigen specifically binding to the anti-ENA antibody which is bound to protein-A-Sepharose. Then, by purifying such an antigen by a well-known method, an RNA in the antigen can be obtained. A cDNA is prepared by utilizing this RNA as a template, and subsequently, transcription product of the relevant cDNA obtained by carrying out the well-known in vitro transcription method can be made as the second RNA. The kinds of the transcription vectors used in the in vitro transcription method is not particularly limited, and the known kind of transcription vector can be used by optionally selecting the known one. It should be noted that prior to in vitro transcription method being carried out, it is preferable that previously the cDNA has been amplified by a PCR method. This procedure is taken in order to increase the yield of the second RNA.

As for the first protein molecule which is substantially the same as the first intracellular non-histone soluble protein , the protein molecule purified from a mammal cell tissue or a mammal cultured cell with a known biochemical technique or the like can be used. Also, in the case where an amino acid sequence of the first intracellular non-histone soluble protein is known in public, a recombinant protein is prepared using a gene coding for this amino acid sequence, which can be made as the first protein molecule. In the case where it is prepared in a large amount, the latter method is particularly preferable. In the case where a recombinant protein is used, it is preferable to use a recombinant protein having a tag portion. Specifically, it is preferable that is made to be a recombinant protein which has been expressed as a fused protein with the tag molecule. It is because the purification is easily performed by utilizing the affinity column using a carrier for specifically binding to the tag portion. As a tag, for example, His-Tag consisted of several histidines, β-D-galactosidase, GST (glutathione S-transferase), thioredoxin, maltose binding protein, Myc, Xpress, FLAG or the like can be used. Among these, His-tag is preferable. Since His-tag is a small molecule, by utilizing this, the tag portion occupying the first protein molecule is made smaller, and thereby it is considered that the influence upon the connectivity between the relevant first protein molecule and the second RNA or the anti-ENA antibody is small. Moreover, it is because it is preferable that the first protein molecule can be resolved into PBS buffer which is a water-borne buffer, Carbonate buffer or Tris buffer or the like. From the viewpoint of the above-described connectivity, it is also preferable that a recombinant protein not having a tag portion is used. Such a recombinant protein can be also prepared by later removing the tag portion of one expressed as a fused protein with the tag molecule.

An RNA protein complex is formed by using the second RNA and the first protein molecule which were prepared as described above. For example, both are added to the water-borne buffer and mixed, thereby binding both to each other. In the case of this, the buffer is not particularly limited. For example, a PBS buffer consisted of the known composition (phosphoric acid buffered physiological saline), carbonate buffer or tris (tris hydroxymethyl aminomethane) buffer is used.

The RNA protein complex which was thus reconstructed and the specimen are reacted. One portion of the components existing in the specimen (anti-ENA antibody) specifically binds to the RNA protein complex, thereby the anti-ENA antibody can be measured by this operation. In this way, since the RNA protein complex (new antigen) which was reconstructed with the second RNA and the first protein molecule and the specimen are reacted, it is possible that only the component having the connectivity to this second RNA, the first protein molecule or a complex of these is specifically measured. This means that only the component binding to the first RNA, the first intracellular non-histone soluble protein or a complex of these is specifically measured since the second RNA and the first protein molecule are substantially the same as the first RNA and the first intracellular non-histone soluble protein contained in the original antigen, respectively.

Even in the case where the second intracellular non-histone soluble protein is contained in an antigen, since an RNA protein complex (antigen) is reconstructed without containing a component corresponding to the relevant second intracellular non-histone soluble protein, with which the specimen is reacted, the component having the connectivity to the relevant second intracellular non-histone soluble protein is not detected in the specimen. Specifically, the detection can be performed without subjecting to the influence of the component having the connectivity to the second intracellular non-histone soluble protein and thereby the measurement with the specificity and high sensitivity is realized.

It should be noted that in the first aspect of the present invention, the second RNA and the first protein molecule corresponding to the first RNA and the first intracellular non-histone protein are prepared respectively and the complex of these (RNA protein complex) is utilized for measurement. Supposing the case where a native antigen is purified from the organism material and using this, a similar measurement is performed. But in this case, the effect of the present invention is not obtained. Specifically, supposing the case where U1RNP is exemplified, since a U1RNP in a native state exists in a state where RNP and Sm are bound to U1snRNA. if the native U1RNP is used as an antigen, an antibody binding to Sm portion in addition to an antibody specifically binding to the U1sn or RNP portion is also detected (measured) as a result. In a method for measurement of the first aspect of the present invention, for example, an RNA protein complex (antigen) not containing Sm is reconstructed and since using this, the measurement is performed, it can be achieved that only the antibody specifically binding to U1snRNA or RNP can be detected without receiving the influence of the antibody binding to Sm portion.

The reaction product generated by reacting an RNA protein complex and the specimen can be directly measured. Specifically, the first aspect of the present invention comprises a method for measuring an anti-ENA antibody which specifically recognizes the antigen containing the first RNA and the first intracellular non-histone soluble protein, which comprises the following steps a)-c).

a) the step in which the complex of the second RNA which is substantially the same as the first RNA and the first protein molecule which is substantially the same as the first intracellular non-histone soluble protein is formed, b) the step in which the specimen is reacted with the complex and, c) the step in which the reaction product generated by the step b) is detected.

Moreover, the first aspect of the present invention comprises a method for measuring an anti-ENA antibody, which specifically recognizes an antigen containing the first RNA, the first intracellular non-histone soluble protein and the second intracellular non-histone soluble protein and whose recognition site is located at any one of the first RNA, the first intracellular non-histone soluble protein or a complex of these, which comprises the following steps a)-c).

a) the step in which the complex of the second RNA which is substantially the same as the first RNA and the first protein molecule which is substantially the same as the first intracellular non-histone soluble protein is formed, b) the step in which the specimen is reacted with the complex and, c) the step in which the reaction product generated by the step b) is detected.

The reaction product generated by reacting the RNA protein complex and the specimen is labeled, and the detection can be also carried out by measuring the labeled amount. In this case, the labeling can be performed, for example, by reacting the antibody (secondary antibody) labeled with the labeling substance with the reaction product generated by the step b) and by binding the relevant secondary antibody to the anti-ENA antibody contained in the reaction product. In this case, as for the secondary antibody, an antibody having the connectivity to the anti-ENA antibody contained in the reaction product is used. In other words, the secondary antibody is appropriately selected corresponding to the kinds of anti-ENA antibodies which are the objects of the measurement. For example, in the case where a human organism fluid (human serum and the like) is used as a specimen, since the anti-ENA antibody is a human antibody, the anti-human immune globulin antibody which has been labeled can be used as a secondary antibody.

As a labeling substance used for labeling, the known fluorescent pigment, enzyme, radioactive material, biotin and the like can be used without particularly being limited if it has a sensitivity sufficient to be capable of being detected. Moreover, a substance having a high electron density such as ferritin, colloidal gold or the like can be also used. As a fluorescent pigment, fluorescein isothiocyanate (FITC), rodamine B isothiocyanate (RITC), phycoerythrin (PE) and the like are listed. As an enzyme, peroxidase, β-D-galactosidase, microperoxidase, alkali phosphatase, acidic phosphatase, cytochrome c and the like are listed. As a radioactive substance, ¹²⁵I, ¹⁴C, ³H and the like are listed.

An RNA protein complex can be used in a solid-phased state by previously being bound to an insoluble support. The stability, the easiness of handling and preservation and the like of the relevant complex are contemplated and the measurement by simple and easy operations can be realized by solid-phasing the RNA protein complex. As an insoluble support, for example, a substance insoluble in water such as a resin such as polystyrene resin, polycarbonate resin, silicon resin, nylon resin and the like, glass, or micelle particle is used, the material is not particularly limited. Moreover, the shape of the insoluble support is neither particularly limited, and a support in a tray shape, in a spherical shape, in a rod shape, in a fiber shape, in a cell shape, in a test tube shape or the like can be employed. The carrying and holding of the complex to this insoluble support is performed by a physical absorption or a chemical absorption.

As a specimen, an organism fluid such as serum, blood plasma. urine, spinal fluid, ascites fluid, pleural fluid or the like is used. It is preferable that the serum is used. In the case where the serum is used, a simple and easy measurement can be realized.

According to the above-described method for measuring, for example, the component in the specimen which specifically binds to the relevant complex, that is, an anti-ENA antibody can be measured by labeling the reaction product after the relevant solid-phased complex is reacted with the specimen and by measuring the labeling amount.

Moreover, the anti-ENA antibody in the specimen can be also indirectly quantified by preparing the anti-ENA antibody as the standard substance capable of binding to the RNA protein complex (hereinafter, referred to as "standard anti-ENA antibody"), then competitively reacting this and the specimen with the RNA protein complex and subsequently measuring the amount of the standard anti-ENA antibody which has been bound, as a result of reaction, to the RNA protein complex. For example, if the standard anti-ENA antibody has been labeled with the labeling substance, the amount of the standard anti-ENA antibody bound to the RNA protein complex is measured.

The standard anti-ENA antibody can be prepared from the serum of the anti-ENA antibody positive, for example, by a known biochemical procedure or the like.

Moreover, as a labeling substance, it is not particularly limited if it has the sensitivity sufficient to be capable of being detected, the known fluorescent pigment, enzyme, radioactive material, biotin or the like can be used. Moreover, a substance having a high electron density such as ferritin, colloidal gold or the like can be also used. As a fluorescent pigment, fluorescein isothiocyanate (FITC), rodamine B isothiocyanate (RITC), phycoerythrin (PE) and the like are listed. As an enzyme, peroxidase, β-D-galactosidase, microperoxidase, alkali phosphatase, acidic phosphatase, cytochrome c and the like are listed. As a radioactive substance, ¹²⁵I, ¹⁴C_{,} ³H and the like are listed.

It should be noted that as a method for directly measuring the reaction product generated by reacting the RNA protein complex and the specimen, for example, a method for diffusing the RNA protein complex and the specimen respectively within a gel and observing the binding of both as a precipitation line can be listed.

As described above, a method for measurement of the present invention is applied to a variety of immunoassays such as ELISA method, fluoroimmunoassay, radioimmunoassay, immune turbidimetry, latex aggregation method, immune precipitation method, double immune diffusion method and the like, and these methods including the step in which the complex of the second RNA and the first protein molecule and the specimen are reacted are included in the present invention.

In the first aspect of the present invention, the determination of whether the specimen is positive or negative or the quantification of the anti-ENA antibody in the specimen can be performed by utilizing what is called the predetermined positive specimen including the anti-ENA antibody of the measuring object as the standard specimen.

The first aspect of the present invention particularly comprises the following methods for measurement:

A method for measuring an anti-U1RNP antibody for specifically recognizing U1RNP consisted of U1snRNA and RNP, characterized in the step of forming a complex of an RNA which is substantially the same with the U1snRNA and a protein molecule which is substantially the same with the RNP is formed, and then reacting the relevant complex and the specimen.

Moreover, the following method is also included in the first aspect of the present invention.

A method for measuring an anti-U1RNP antibody which specifically recognizes the U1RNP consisted of U1snRNA and RNP, which comprises the following steps A)-C):
A) a step of forming a complex of an RNA which is substantially the same as the U1snRNA and a protein molecule which is substantially the same as the RNP,
B) a step of reacting the specimen with the foregoing complex, and
C) a step of detecting a reaction product generated by the step B).

In the step C), after the reaction product generated by the step B) has been labeled, the detection of the reaction product may be performed by measuring the labeled amount.

The second aspect of the present invention is a complex consisting of the following constitution:

Specifically, a complex used for measuring an anti-ENA antibody for specifically recognizing an antigen containing the first RNA and the first intracellularnon-histone soluble protein, which comprises the second RNA which is substantially the same as the first RNA and the first protein molecule which is substantially the same as the first intracellular non-histone soluble protein. A complex consisted of the following constitution is included in the second aspect of the present invention. A complex used for measuring an anti-ENA antibody, which specifically recognizes an antigen containing the first RNA, the first intracellular non-histone soluble protein and the second intracellular non-histone soluble protein and whose recognition site is located at any one of the RNA, the first intracellular non-histone soluble protein or a complex of these, that is, a complex comprising the second RNA which is substantially the same as the foregoing first RNA and the first protein molecule which is substantially the same as the foregoing first intracellular non-histone soluble protein.

A complex of the second aspect of the present invention can be used for a method for measuring an anti-ENA antibody which is the first aspect of the above-described present invention. In other words, an anti-ENA antibody which is a component binding to the relevant complex in the specimen can be measured by reacting the specimen with the relevant complex.

Since the respective factors of the first RNA, the first intracellular non-histone soluble protein, the second intracellular non-histone soluble protein, antigen, anti-ENA antibody, the second RNA which is substantially the same as the first RNA, the first protein molecule which is substantially the same as the first intracellular non-histone soluble protein and a complex of these have been set forth in the aspect of the above-described present invention, the description is omitted.

A complex consisting of the following constitution is particularly included in the second aspect of the present invention. Specifically, a complex consisting of an RNA which is substantially the same as U1sn and a protein molecule which is substantially the same as an RNP.

The third aspect of the present invention is a solid-phased antigen in which a complex in the second aspect of the above-described present invention is bound to an insoluble support. Specifically, it is a solid-phased antigen for measuring an anti-ENA antibody, which is constituted by binding the complex of the second RNA and the first protein molecule to the insoluble support.

A solid-phased antigen which is the third aspect of the present invention can be used for a method for measuring an anti-ENA antibody which is the first aspect of the present invention as similar to the case of the above-described second aspect. In other words, an anti-ENA antibody which is a component binding to the relevant solid-phased antigen in the specimen can be measured by reacting the specimen with the relevant solid-phased antigen. The stability, the easiness of handling and preservation and the like of the relevant complex are contemplated and the measurement performed by simple and easy operations is capable of being realized by solid-phasing the complex of the second RNA and the first protein molecule.

Since the attribution of the insoluble support and the method for binding the complex to the insoluble support have been described in detail in the first aspect of the above-described present invention, the description is omitted.

A solid-phased antigen for measuring an anti-U1RNP antibody which is constituted by binding the complex consisted of an RNA which is substantially the same as U1snRNA and a protein molecule which is substantially the same as RNP is particularly included in the third aspect of the present invention.

The fourth aspect of the present invention is a kit for measuring an anti-ENA antibody consisting of the following constitution: specifically, a kit for measuring an anti-ENA antibody which specifically recognizes an antigen containing the first RNA and the first intracellular non-histone soluble protein, which comprised a solid-phased antigen which has a solid-phased complex of the second RNA which is substantially the same as the first RNA and the first protein molecule which is substantially the same as the first intracellular non-histone soluble protein to an insoluble support, an anti-human immune globulin antibody and the standard specimen containing the anti-ENA antibody. Moreover, a kit for measuring an anti-ENA antibody, which specifically recognizes an antigen containing the first RNA, the first intracellular non-histone soluble protein and the second intracellular non-histone soluble protein and whose recognition site is located at any one of the first RNA, the first intracellular non-histone soluble protein or a complex thereof, which comprises a solid-phased antigen which has solid-phased a complex of the second RNA which is substantially the same as the first RNA and the first protein molecule which is substantially the same with the foregoing first intracellular non-histone soluble protein to the insoluble support, an anti-human immune globulin antibody and the standard specimen containing an anti-ENA antibody.

When a kit for measuring an anti-ENA antibody which is the fourth aspect of the present invention is used, the simple and easy measurement of the anti-ENA antibody is capable of being realized. Moreover, the relevant kit can be used for a method for measurement of the above-described present invention.

Since the respective factors of the first RNA, the first intracellular non-histone soluble protein, the second intracellular non-histone soluble protein, antigen, anti-ENA antibody, the second RNA, the first protein molecule, a complex thereof, an insoluble support and a solid-phased antigen have been set forth in the first and third aspects of the above-described present invention, the description is omitted.

The kinds of anti-human immune globulin antibodies are not particularly limited, and known ones can be used. Moreover, as for the anti-human immune globulin antibody, an antibody which has been labeled can be used.

As an anti-human immune globulin antibody which has been labeled, for example, peroxidase labeled anti-human IgG (γ chain) antibody code number 208, anti-human IgA (α chain) antibody code number 210, and anti-human IgM (µ chain) antibody code number 212 (made by Medical Biological Institute, Co. , Ltd.), which are commercially available, can be used. Moreover, as a labeling substance, it is not particularly limited if it has the sensitivity sufficient to be capable of be detected, the known fluorescent pigment, enzyme, radioactive material, biotin or the like can be used. Moreover, a substance having a high electron density such as ferritin, colloidal gold or the like can be also used. As a fluorescent pigment, fluorescein isothiocyanate (FITC), rodamine B isothiocyanate (RITC), phycoerythrin (PE) and the like are listed. As an enzyme, peroxidase, β-D-galactosidase, microperoxidase, alkali phosphatase, acidic phosphatase, cytochrome c and the like are listed. As a radioactive substance, ¹²⁵I, ¹⁴C, ³H and the like are listed.

As the standard specimen including an anti-ENA antibody, an organism fluid such as serum, blood plasma, urine, spinal fluid, ascites fluid, pleural fluid or the like of the anti-ENA antibody being positive is used. It is preferable that the serum is used.

A kit having the following constitution is particularly included in the fourth aspect of the present invention. Specifically, it is a kit for measuring an anti-U1RNP antibody, which comprised a solid-phased antigen which has a solid-phased complex of an RNA which is substantially the same as U1snRNA and a protein molecule which is substantially the same as an RNP to an insoluble support, an anti-human immune globulin antibody and the standard specimen containing an anti-U1RNP antibody.

Also in this case, a labeled antibody labeled can be used as an anti-human immune globulin antibody.

### (Example 1) Preparation of U1snRNA by in vitro transcription method

U1snRNA was prepared by an in vitro transcription method using a combination of pGEM-4Z (made by Promega, Co., Ltd.) which is a transcription vector, and RiboMAX Large Scale RNA Production Systems (made by Promega, Co., Ltd.).

### (1-1) Preparation of U1DNA

A transcription vector capable of transcribing U1snRNA was prepared by cloning U1DNA which is to be a template for transcribing U1snRNA and then incorporating it into pGEM-4Z vector. The concrete operations were carried out as follows:

First, 2 mg of protein A-Sepharose (made by Amersham PharmaciaBiotech, Co., Ltd.), 500 µl of IPPbuffer (10 mM Tris-HCl pH 8.0, 500 mM NaCl, 0.1% NP-40) and 20 µl of the serum of a patient with the anti-U1RNP being positive were mixed in 1.5 mL micro-centrifugal tube, and reacted while gently shaking at 4°C over night, and thereby the anti-U1RNP antibody was absorbed by the protein A-Sepharose. Next, the centrifugal treatment was carried out (15,000 rpm, 1 min., and 4°C), and the supernatant was discarded. After 500 µl of IPP buffer was added to a pellet and it was well agitated by pipette operation, and after the centrifugal treatment was carried out again(15,000 rpm, 1 min., and 4°C), the supernatant was discarded. The marginal protein non-specifically absorbed to the protein A-Sepharose was removed by repeating this operation three times. 400 µl of NET-2 buffer (50 mM Tris-HCl pH 7.5, 150 mM NaCl) was added to the pellet obtained by the above-described operation, and further, by reacting a compound to which 200 µl of HeLa cell extract (preparation method of HeLa cell extract, for example, after NET-2 buffer was added to the HeLa cell and well suspended, the cell was broken by carrying out the supersonic treatment, and insoluble matters is removed by carrying out the centrifuge separation) was added at 4 °C for 2 hours while gently shaking, U1snRNA-RNP-Sm complex in the HeLa cell extract was trapped (bound) by the anti-U1RNP antibody absorbed on the protein A-Sepharose.

Next, the centrifugal treatment was carried out (15,000 rpm, 1 min., and 4°C) for the purpose of removing the marginal protein, and the supernatant was discarded. 500 µl of NET-2 buffer was added to the obtained pellet, and well agitated by pipette operation. After this was centrifuge-treated (15,000 rpm, 1min., and 4°C) again, the supernatant was discarded. The washing effect was enhanced by repeating this operation 5 times. After the final centrifugal treatment was carried out (washing), the supernatant was discarded, and 300 µl of NET-2 buffer, 15 µl of 10%SDS and 30 µl of 3M sodium acetate (pH 5) were sequentially added. Furthermore, after 300 µl of phenol/chloroform mixture was added, sufficiently agitated, and the centrifugal treatment was carried out (15,000 rpm, 5 min., and 4°C). The upper layer obtained as a result was transferred to another Eppen tube, and made it as U1snRNA solution.

After 900 µl of cold ethanol was added to the U1snRNA solution, it was cooled by standing at -80°C for one hour. Subsequently, the centrifugal treatment was carried out (15,000 rpm, 10 min., and 4°C), and U1snRNA was precipitated.

Next, U1snRNA in a pellet state was resolved in 20 µl of a sterile water free of ribonuclease, a DNA primer (5'-CAGGGGAAAGCGCGAACGCAGTCCCCCACTA-3')( SEQ ID No.1) capable of pairing with the 3' side of U1snRNA added, and aheteroduplex of RNA-DNA was prepared by making a reverse transciptase act. Subsequently, by making this heteroduplex as a template, a PCR reaction was performed by the known method using (5'-ATACTTACCTGGCAGGGGAGATACCATGATCA-3') (SEQ ID No.2) as an Upper PCR primer and a DNA primer used for the above-described reverse transcriptase reaction as a Lower PCR primer, and U1DNA fragment (SEQ ID No.3) was obtained.

### (1-2) Acquisition and purification of U1snRNA by in vitro transcription method

It was made a vector capable of transcribing U1snRNA by inserting the sequence in which linkers of EcoRI and BamHI are added to the U1DNA fragment obtained in the above-mentioned description by a known method (for example, see Genetic Engineering Experiment Note <Basis of DNA handling and subcloning>, p.114, Yodosha, 1997) between EcoRI and BamHI of multicloning sites under the control of a promoter (SP6 prommoter) of SP6 RNA polymerase of pGEM-4Z (hereinafter, this vector is referred to as "pGEM-U1snRNA vector"). Next, in vitro transcription method was carried out using a pGEM-U1snRNA vector by the following method, and thereby U1snRNA was prepared.

First, a pGEM-U1snRNA vector was purified at a high purity according to the known cesium chloride method. Subsequently, in order to prevent ribonuclease from being mixed, it was treated at 37°C for 30 minutes with Proteinase K (100 µg/mL), SDS(0.5%), 50 mM Tris-HCl (pH 7.5), and 5 mM CaCl₂. Subsequently, deproteinization treatment was performed with phenol/chloroform mixture, and subsequently pGEM-U1snRNA was precipitated by adding ethanol. The highly purified pGEM-U1snRNA obtained as a result of it was treated with a restriction enzyme BamHI according to a known procedure, and made it in a linear shape. By using this linear pGEM-U1snRNA as a template, the transcription reaction was performed using RiboMax Large Scale RNA production Systm-SP6 (made by Promega, Co., Ltd.). The transcription reaction was performed by mixing 50 µL of the linear pGEM-U1snRNA (1 µg/mL), 100L of Sp6 RNA polymerase 5 times condensed buffer, 25 µL of rATP (100 mM), 25 µL of rGTP (100 mM), 25 µL of rCTP (100 mM), 25 µL of rUTP (100 mM), 200 µL of sterile water free of nuclease and 50 µL of SP6 Enzyme Mix, and by incubating 500 µL of the total volume at 37°C for 4 hours. The other operation procedure and the like were performed according to the operation explanatory note attached to RiboMax Large Scale RNA production System-SP6.

Subsequently, U1snRNA was purified from the reaction termination liquid by the following method. At the beginning, one unit of DNase (made by Promega, Co., Ltd.) per 1 µg of linear pGEM-U1snRNA (template DNA) was added, and the DNase treatment was performed by reacting it at 37°C for 15 minutes. After the reaction was terminated, 500 µL of phenol/chloroform/isoamyl alcohol (ratio of the respective liquids are in turn 25:24:1) mixture which has been saturated with TE buffer, pH 4.5 was added and well agitated, and subsequently, after the centrifugal treatment was carried out (15,000 rpm, 5 min., and 4°C), the upper layer was transferred to a new Eppen tube. By this operation, pGEM-U1snRNA (template DNA) which has been DNase-treated and unreacted rNTPs were removed. Subsequently, 50 µL of sodium acetate (pH 5.2) was added, and further, 500 µL of isopropanol was added, and after then it was standing and cooled for 5 minutes on ice, U1snRNA was precipitated by centrifuging (15,000 rpm, 10 min., and 4°C). After the precipitated U1snRNA was washed with cold 70% ethanol and dried under the vacuum, it was resolved in 500 µL of a sterile water free of nuclease(purified U1snRNA) .

After the purified U1snRNA obtained as a result of the above-described procedure was diluted 300 times, its concentration was calculated by the measurement of the absorbance in the wavelength of 260 nm, and the yield was found. As a result of this, about 2-3 mg per 500 µL of purified U1snRNA was obtained in the purified stage. It should be noted that this calculation was performed by defining the absorbance (A260) = 1.0 in the wavelength of 260 nm corresponding to RNA 40 µg/mL. The calculation was performed.

### (Example 2) Preparation of RNP

3 species of recombinant RNPs to which histidine tags were fused (His-tagged RNP 70k (68 kDa), His-tagged RNPA (35 kDa) and His-tagged RNPC (23 kDa)) were prepared by the following method.

### (2-1) Preparation of expression system of His-tagged RNP 70k (68 kDa) and purification of His-tagged RNP 70k

An insect cell expression system was used for expression of His-tagged RNP 70k (68 kDa). Concretely, it was carried out by utilizing the expression system made by Pharmingen, Co., Ltd. (BaculoGold Baculovirus system). Hereinafter, the outline will be described.

First, by using the cDNA prepared from HeLa cell as a template, RNP 70k gene was amplified by the known PCR method using Upper PCR primer (5'-ATGACCCAGTTCCTGCCGCCCAACCTTCTG-3') (SEQ ID No.4) and Lower PCR primer (5'-CTCCGGCGCAGCCTCCATCAAATACCCATT-3') (SEQ ID No.5), a DNA fragment (SEQ ID No.6) to which DNA sequence to be 6 pieces of His was added on 3'-side was prepared. The sequence in which linkers of EcoRI and BamHI are added to this DNA fragment was inserted between EcoRI and BamHI in the multicloning sites of pVL1393 vector (made by Pharmingen, Co., Ltd.) by the known method (for example, see Gene Engineering Experiment Note <Basis of DNA handling and subdoning>, p.114, Yodosha, 1997), and it was made RNP 70k expression vector (referred to as pVL/RNP 70k). This pVL/RNP 70k was mixed with Baculo Gold Linearized Baculovirus DNA (made by Pharmingen, Co, Ltd.), and transfected into SF9 cell by performing a homologous recombination. The plaqueassay was performed using transfected SF9 cell, ones that transparent plaque was generatedwere selected, and by amplifying this, a virus liquid was prepared.

On the other hand, 8 L of TMN-FH medium was inputted into a spinar flask, within this, High Five cell was suspended and cultured at 27°C and at 70 rpm until the number of the cells became 3-7 x 10⁵ cells/mL. After it has been confirmed that it became the predetermined number of cells, virus infection was made by adding the above-described virus liquid so as to be m.o.i. (multiplicity of infection) = 5, and further, was suspended and cultured at 27°C at 70 rpm for 3 days. The High Five cells were collected by performing the centrifugal treatment of the cultured liquid obtained in this way, 20 mL of lysis buffer (1% Triton X-100, 10 mM NaF in PBS) per 3 g of the cells was added and well suspended, and stood on ice for 30 minutes. Subsequently, the centrifugal treatment was carried out, the broken cells were collected as the precipitated matter. 8M urea (in PBS) was added to this precipitated matter, and the precipitation was made solubilized. His-tagged RNP 70k was purified from the insect cell liquid obtained in this way as follows: first, His-tagged RNP 70k was absorbed by previously balancing Chelating Sepharose FF column (made by Pharmacia, Co., Ltd.) which carried and held nickel ion and by adding the above-described insect cell liquid to this. After the non-specifically bound component was washed and removed, the His-tagged RNP 70k which was absorbed in the column was eluted using PBS buffer (pH 8) containing imidazole. At this time, it was eluted while changing the imidazole concentration from 10 to 500 mM, and the fraction containing His-tagged RNP 70k was obtained (purified His-tagged RNP 70k).

### (2-2) Preparation of expression system of His-tagged RNPA(35 kDa) and purification of His-tagged RNPA

The expression system of His-tagged RNPA protein can be constructed from the combination of the expression vector pET28a (+) and E. coli BL21 (DE3), and the system (pET System) made by Novagen, Co., Ltd. was utilized. Hereinafter, the outline of the operation method will be set forth.

First, by making cDNA prepared from HeLa cell as a template, the gene coding RNPA was amplified by the known PCR method using Upper PCR primer (5' -ATGGCAGTTCCCGAGACCCGCCCTAACCAC- 3, ) (SEQ ID No.7) and Lower PCR primer (5'-CTTCTTGGCAAAGGAGATCTTCATGGCGT-3')(SEQ ID No.8), a DNA fragment to which linkers of NcoI and XhoI were added (IDENTIFICATION SEQUENCE NO: 9) by the known method (for example, see Gene Engineering Experiment Note <Basis of DNA handling and subcloning>, p.114, Yodosha, 1997), was inserted between NcoI site and XhoI site in the multicloning sites of pET28a (+) and it was made RNPA expression vector (referred to as pET/RNPA). The expression product of this expression vector is a product in which 6 pieces of His (histidine) were fused to the C-terminal of RNPA. This pET/RNPA was transformed into E. coli BL21 (DE3) and the expression system of His-tagged RNPA was constructed.

Next, E. coli BL21 (DE3) transformed with pET/RNPA was cultured while shaking in 120 mL of a LB culture medium containing 50 µg/mL of kanamycin at 37°C, at 200 rpm for 16 hours. This culture medium was made the seeded mother culture medium. The present culture was performed as follows: specifically, a LB culture medium containing 6 L of 1% glucose and 50 µg/mL of kanamycin was prepared in a jar culture apparatus of 10 L scale, 120 mL of the seeded culture medium was added to the jar culture apparatus, and it was cultured until OD600 became 5-8 while agitating at 37°C. Subsequently, IPTC (isopropyl-thio- β -D-galactoside) was added so that the concentration became 1 mM, and further cultured for 2 hours. Next, E. coli were collected by centrifuging the culture medium, 2% Tween-20, 0.1 mM PMSF and PBS 300 mL containing 1µg/mL Leupepsin were added and suspended and E. coli were broken. Subsequently, the broken supernatant was harvested by performing the centrifugal treatment. The E. coli extracted liquid obtained by the above-described operation was added to the previously balanced Chelating Sepharose FF column (made by Pharmacia, Co., Ltd.) which has carried and held the nickel ion, and His-tagged RNPA in E. coli extracted liquid were absorbed. After the component non-specifically absorbed on the column was washed and removed, His-tagged RNPA which has absorbed on the column was eluted with PBS buffer (pH 8) containing imidazole. At the time, it was eluted while changing imidazole concentration from 10 to 500 mM, and the fraction containing His-tagged RNPA was obtained (purified His-tagged RNPA).

### (2-3) Preparation of expression system of His-tagged RNPC (23 kDa) and purification of His-tagged RNPC

As an expression system of His-tagged RNPC, the expression system which is the same with the above-described His-tagged RNPA was used. That a DNA fragment for being inserted between NcoI and XhoI of the multicloning sites of the expression vector pET28a (+) is not a DNA fragment coding RNPA, but a gene fragment (SEQ ID No.10) coding RNPC is only one different point from the above-described DNA fragment. The PCR primer used for amplifying this RNPC gene by a PCR are Upper PCR primer (5'-ATGCCCAAGTTTTATTGTGACTACTGCGAT-3') (SEQ ID No.1l and Lower PCR primer (5'-TCTGTCTGGTCGAGTCATTCCGGGCCGAGT-3') (SEQ ID No.12). Moreover, the purification of His-tagged RNPC was also performed by the same purification method with the above-described His-tagged RNPA.

### (Reference Experiment Example 1) Consideration of reactivity between U1snRNA and dissociation specimen

### (1) Measurement of anti-U1RNP antibody by DID method

DID method (Double Immune Diffusion method) is also referred to as alias Ouchterlony method, and it is a method which utilizes the principle that after several holes were opened on agar plate, when the antigen and the antibody are diffused opposingly from separate holes, the precipitation lines are formed at the time when the concentrations of both are at the optimum level.

The measurement of anti-U1RNP antibody performed by a DID method was carried out using ENA-1 test (made by Medical Biological Institute, Co., Ltd.). The measurement was carried out according to the operation explanatory note attached to the ENA-1 test.

First, 20 µL of lyophilized crude antigen (crude U1RNP antigen) extracted from rabbit thymus which has been resolved in 100 µL of a sterile water was placed on the center hole of the agar plate, and 20 µL each of anti-U1RNP antibody positive control serum and the specimen serum were alternately placed on the opened hole around the center hole. The precipitation line can be observed after it was reacted at room temperature exceeding over one night. The determination of whether the anti-U1RNP antibody exists (positive) in the specimen, or does not exist (negative) in the specimen is performed by whether the precipitation line which is formed by binding anti-U1RNP antibody and anti-Sm antibody contained in the positive control and the component in the crude U1RNP antigen and the precipitation line which is formed by binding the specimen and the component in crude U1RNP antigen are fused or not. Usually, as for the precipitation line by positive control, two lines can be observed, the precipitation line by the anti-U1RNP antibody is seen the near side of the center section, and the precipitation line by the anti-Sm antibody is seen on the outer side of the other precipitation line.

### (2) Measurement of anti-U1RNP antibody by ELISA method (conventional ELISA method) using only RNP (ribonucleoprotein)

Three species of recombinant RNPs (Recombinant His-tagged RNP) obtained in Example 2 were added to PBS so that RNP 70k; 0.55 µg/mL + RNPA; 0.27 µg/mL + RNPC; 0.18 µg/mL and then the total of these became 1 µg/mL, and immediately after that, 100 µL/well was dispensed to each well of 96 wells micro-titer plate (Maxisorp Nunc). The recombinant RNP was absorbed on the plate by leaving at 4°C over night. The reaction solution was removed from each well, subsequently, PBS 200 µL was added to each well and left for about 10 seconds. Subsequently, PBS in each well was removed. These washing operations were repeated twice, and RNP which has not absorbed on the plate was removed. Furthermore, 200 µL of blocking buffer (1% BSA/PBS) was added to each well, and the blocking was performed by standing at 4°C overnight. Subsequently, the blocking buffer was removed from each well, and each well was sufficiently dried and made it a plate for measuring ELISA.

Using the plate for ELISA measurement obtained in this way, the anti-U1RNP antibodies in the respective specimen were measured using the serum of a patient being positive or the serum of a patient being negative by the DID method of Example 2 (2-1) and the serum of the healthy person. In the measurement, 100 µL to which each serum was diluted 100 times with the buffer for diluting the specimen was added to each well of the plate for ELISA measurement, and the primary reaction was carried out by standing at 25°C for one hour. Subsequently, after the reaction solution was removed from each well, each well was sufficiently washed with PBS containing 1% Tween-20. Subsequently, 100 µL of the labeled antibody liquid containing anti-human IgG antibody which has been labeled with peroxidase was added to each well, and the secondary reaction was carried out by standing 25°C for one hour. After the reaction solution was removed, each well was sufficiently washed with PBS containing 1% Tween-20, subsequently, 100 µL of enzyme matrix liquid containing tetramethylbenzidine and hydrogen peroxide was added to each well. After the enzyme reaction was carried out at 25°C for 30 minutes, 100 µL of 1N sulfuric acid was added to each well and color developed and 450 nm of absorbance was measured by a spectrophotometer.

As a result of this, some of the specimens, which had been positive by the DID method, were determined as negative by the ELISA method. The relevant specimens (dissociation specimen) were used for consideration in the following (3). The specimens determined as negative by the DID method were all determined as negative also by the ELISA method. As for the specimens of healthy persons, all of them were determined as negative.

### (3) Consideration of reactivity between the specimens determined as positive by DID method and determined as negative by ELISA method (dissociation specimens) and U1snRNA

By performing the comparison between the above-described (1) and (2), the reactivity between the specimens which have been determined as positive by the DID method and determined as negative by the ELISA method using recombinant RNP (dissociation specimens) and the U1snRNA obtained in (1-2) of Example 1 was considered by the immune precipitation method and the ELISA method.

As for an immune precipitation method, it was carried out on the basis of the procedure developed by Mark S. Forman et al. (Arthritis and Rheumatism, Vol. 28, No.12, 1356-1361, 1985). Instead of HeLa cell extract in the procedure by Mark S. Forman et al., U1snRNA obtained by an in vitro transcription method in (1-2) of Example 1 was used. Concretely, the following operations were carried out.

First, 2 mg of protein A-Sepharose (made by Amersham Pharmacia Biotech, Co., Ltd.), 500 µL of IPP buffer (10 mM Tris-HCl pH 8.0, 500 mM NaCl, 0.1% NP-40) and 20 µl of serum of the dissociation were mixed in 1.5 mL micro-centrifugal tube, and reacted while gently shaking at 4°C over night, and the anti-U1RNP antibody was absorbed on the protein A-Sepharose. Next, the centrifugal treatment was carried out (15,000 rpm, 1 mm., and 4°C), and the supernatant was discarded. After 500 µL of IPP buffer was added to a pellet and it was well agitated by pipette operation, and after the centrifugal treatment was carried out again (15,000 rpm, 1 min., and 4°C), the supernatant was discarded. The marginal protein non-specifically absorbed to the protein A-Sepharose was removed by repeating this operation three times. 400µl of NET-2 buffer (50 mM Tris-HCl pH 7.5, 150 mM NaCl) was added to the pellet obtained by the above-described operation, and further, U1snRNA was bound to anti-U1RNP antibody in the dissociation specimen absorbed on the protein A-Sepharose by reacting a compound to which 25 µL of U1snRNA (5 mg/mL) prepared by an in vitro transcription method in (1-2) of Example 1.

Next, the centrifugal treatment was carried out (15,000 rpm, 1 min. , and 4°C) for the purpose of removing the marginal U1snRNA, and the supernatant was discarded. 500 µL of NET-2 buffer was added to the obtained pellet, and well agitated by pipette operation. After this was centrifuge-treated (15,000 rpm, 1min., and 4°C) again, the supernatant was discarded. The washing effect was enhanced by repeating this operation 5 times. After the final centrifugal treatment (washing) was carried out, the supernatant was discarded, and 300 µl of NET-2 buffer and 15 µl of 10%SDS and 30 µl of 3M sodium acetate (pH 5) were sequentially added. Furthermore, after 300 µl of phenol/chloroformmixturewas added, sufficiently agitated, and the centrifugal treatment was carried out (15,000 rpm, 5 min., and 4°C). The upper layer obtained as a result of this was transferred to another Eppen tube, after 900µL of cold ethanol was added to this solution, it was cooled by standing at -80°C for one hour. Subsequently, the centrifugal treatment was carried out (15,000 rpm, 10 min., and 4°C). The obtained pellet was resolved in a buffer, it was subjected to electrophoresis within 10% polyacrylamide gel containing 7M urea. The U1snRNA was confirmed by subjecting gel after the electrophoresis to silver dying (silver stain plus kit; made by Bio-Rad).

The results on the dissociation specimens(14 specimens, serum 1-serum 14) are shown in Fig. 1. Fig. 1 is a photograph showing a gel after being dyed with silver. The samples prepared from the serums 1-14 were separately flowed in lanes 3-16. As a control, in lane 1, a U1snRNA (positive control) prepared by an in vitro transcription method of (1-2), and in lane 2, the sample prepared from serum of the healthy person (negative control) were separately flowed. The arrow shows the band location of the U1snRNA As shown in Fig. 1, it is understood that the bands of U1snRNA are observed in one portion of lanes (lanes 6, 8). Specifically, it was indicated that the specimens having the reactivity with U1snRNA in the dissociation specimens are only one portion of the specimens.

Since a method for measuring using ELISA method is similar to the method for measuring an anti-U1RNP antibody by the conventional ELISA method of the above-described (2), the description is omitted, here, only the solid-phasing method will be described below.

First, 100 µL each of the liquid diluted with 150 mM Tris, 0.05% Tween-20 so that the concentration of methylated BSA became at the level of 50 µg/mL was added to each well of the 96 wells micro-titer plate (Maxisorp Nunc), and after leaving at 25°C for 2 hours, the reaction solution was removed from each well. Subsequently, 100 µL each of the solution diluted with 10 mM Tris, 0.05% Tween-20, 1 mM EDTA so that the concentration of U1nRNA became 20 µg/mL obtained in (1-2) of Example 1 was added to each well and left at 4°C over night, thereby absorbing U1snRNA on the plate via methylated BSA. The reaction solution was removed from each well, and subsequently, 200 µL of the blocking buffer (1%BSA/PBS) was respectively added to each well, left at 4°C over night, thereby performing the blocking. After the blocking buffer was removed from each well, it was made a plate for ELISA measurement by sufficiently drying it.

The reactivity with U1snRNA was examined on the dissociation specimens (serums 1-14) using the plate for ELISA measurement prepared in this way. As a result of this, the reactivity (positive) was recognized only in one portion of the specimens.

The results of the above-described immune precipitation method and ELISA method were compiled in the table of Fig. 2. It should be noted that the results of measurement of each dissociation specimens by DID method in the above-described (1) and the conventional ELISA method (ELISA method using the plate on which only RNP protein is solid-phased) in the abone-described (2) are also indicated together with the results of the immune precipitation method and ELISA method.

As shown in Fig. 2, the specimens having the reactivity against U1snRNA are two specimens (serum 4 and serum 6) among the dissociation specimens 14. These results indicate that the existence of antibody for recognizing U1snRNA in some of the dissociation specimens, and at the same time, it suggests that among the dissociation specimens, anti-U1RNP antibodies which do not recognize a single U1snRNA or a single RNP exist. Specifically, it was suggested that, in addition to the anti-U1RNP antibodies which individually recognize U1snRNA and three species of RNP proteins, there exists the anti-U1RNP antibody which recognizes U1snRNA-RNP protein complex.

### (Reference Experiment Example 2) Consideration of reactivity of U1RNP antigens in DID method

The reactivity between the antigen that the crude U1RNP antigen used for the DID method of (1) of Reference Experiment Example 1 was treated with RNaseA and RNaseT1, and the dissociation specimens which have been determined as positive by the DID method of the same Experiment Example (1) and determined as negative by the conventional ELISA method of the same Experiment Example (2) was examined by the DID method.

The RNase treatment of the crude U1RNP antigen was carried out in the following manner: the crude U1RNP antigen which is the lyophilized crude antigen extracted from rabbit thymus contained in ENA-1 test (made by Medical Biological Institute, Co., Ltd.) was resolved in 90 µL of sterile water, and to this, 5 µL of RNaseA (Code313-01461; Nippon Gene) and 5 µL of RNaseT1 (Code 109193; Berlinger Manheim) were added, and reacted at 370C for 30 minutes. Moreover, the measurement by the DID method was carried out similar to the method described in (1) of Reference Experiment Example 1. As a result, no precipitation line for indicating the existence of the anti-U1RNP antibody was observed in these dissociation specimens. Specifically, in all of the dissociation specimens, the lowering or deletion of the reactivity was recognized by performing the RNase treatment to the crude U1RNP antigen. This suggests that RNP in the crude U1RNP antigen exists in a state of forming a complex with U1snRNA.

### (Example 3) Preparation of complex solid-phased antigen (plate for ELISA measurement) of U1snRNA and RNP

### (3-1) Preparation of complex of U1snRNA and RNP

The three species of His-tagged RNPs (His-tagged RNP 70k, His-tangged RNPA and His-tagged RNPC) obtained in Example 2 were added to PBS so that the total amount became 1 µg/mL (His-tagged RNP 70k; 0.55 µg/mL, His-tagged RNPA; 0.27 µg/mL, and His-tagged RNPC; 0. l8 µg/mL). Furthermore, after UlsnRNA obtained in Example 1 was added, by incubating at 37°C for 5 minutes, a complex of U1snRNA and three species of His-tagged RNPs were prepared. At this time, the addition concentration of U1snRNA was changed in the range from 0 to 25 µg/mL (respective concentrations of 0, 5, 10, 15, 20, 25 µg/mL), the antigen solutions whose U1snRNA addition concentration were 0, 5, 10, 15, 20 and 25 µg/mL respectively, were prepared. It should be noted that the antigen solution was used for the preparation of the following complex solid-phased antigens immediately after the incubation was terminated.

### (3-2) Preparation of complex solid-phased antigen (plate for ELISA measurement)

100 µL each of the antigen solution which are different in U1snRNA addition concentration obtained in the above-described (3-1) was dispensed to each well of the 96 wells micro-titer plate (made by Maxisorp Nunc, Co., Ltd.), and left at 4°C over night, thereby absorbing U1snRNA-RN protein complex on the plate. Subsequently, the antigen solution was removed from each well, and 200 µL of the PBS buffer was added to each well. After leaving for about 10 seconds, the PBS buffer was removed from each well. The unabsorbed U1snRNA and RNP or the like were removed by repeating this washing operation twice. Furthermore, the blocking buffer (1%BSA/PBS) was added to each well, and left at 4°C over night. Subsequently, the blocking buffer was removed from each well, it was made a plate for ELISA measurement by sufficiently drying it.

### (Example 4) Construction of kit for ELISA measurement

A kit for measuring anti-U1RNP antibody was constructed by combining a plate for ELISA measurement in which U1snRNA and three spices of His-tagged RNPs (His-tagged RNP 70k, His-tagged RNPA and His-tagged RNPC) prepared according to Example 3 were absorbed, anti-human IgG antibody in which peroxidase was labeled (made by Medical Biological Institute, Co., Ltd.) and the serum of a patient whose anti-U1RNP antibody is positive.

### (Example 5) Measurement of anti-U1RNP antibody in specimen by ELISA method

The following anti-U1RNP antibodies in the specimens were measured using the plate for ELISA measurement obtained in Example 3. It should be noted that each specimen was measured using wells having different U1snRNA concentration in the antigen solutions (i.e., wells different in absorbing amount of U1snRNA or the complex of U1snRNA and RNP) at the time when the antigen is absorbed on the plate for ELISA measurement.

The specimens made as the measurement object are the 12 serum specimens(dissociation specimens) which had been recognized as positive by the DID method in the reference experimental example 1 and which had been recognized as negative by the conventional ELISA method (i.e., a method for observing the binding an antigen (solid-phased antigen) in which only three species of recombinant RNPs were fixed on the microplate and anti-U1RNP in the specimen), the 8 serum specimens(positive specimens) which were determined as positive by both of the DID method and the conventional ELISA method and 21 specimens (healthy persons' specimens) consisted of the healthy persons' serum.

First, the primary reaction was carried out by adding 100 µL of the solution to which each specimen was diluted 100 times with the buffer for reaction to each well of the plate for ELISA measurement and by standing at 25°C for one hour. After the reaction solution was removed from each well, each well was sufficiently washed with PBS containing 1% Tween-20. Subsequently, the secondary reaction was carried out by adding 100 µL of the labeled antibody solution containing anti-human IgG antibody which had been labeled with peroxidase (made by Medical Biological Institute, Co., Ltd.) to each well and by standing at 25°C for one hour. After the reaction solution was removed from each well, each well was sufficiently washed with PBS buffer containing 1% Tween-20. Subsequently, 100 µL of enzyme matrix liquid containing tetramethylbenzidine and hydrogen peroxide was added to each well, and the enzyme reaction was carried out at 25°C for 30 minutes. Subsequently, after 100 µL of 1N sulfuric acid was added to each well and the enzyme reaction was quenched, 450 nm of absorbance was measured by a spectrophotometer.

The results of the above-described measurements are shown in the table of Fig. 3 the graph of and in Fig. 4. In Fig. 3 and Fig. 4, the dissociation specimens are shown as the dissociation 1-dissociation 12. Similarly, the positive specimens are shown as the positive 1-positive 8, and the healthy persons' specimens are shown as the healthy person 1-healthy person 21, respectively. In the table of Fig. 3, the results which have been measured using wells being different in U1snRNA concentration in the antigen solution at the time when the antigen is absorbed on the plate for ELISA measurement are compiled and shown.

From the graph of Fig. 4, it is understood that in the dissociation specimens determined as positive by the DID method and determined as negative by the conventional ELISA method, anti-U1RNP antibody in the specimen was detected in larger amount in the wells prepared by adding the antigen solution where U1snRNA addition concentration had been increased. Specifically, the reactivity (detection sensitivity) was significantly enhanced by utilizing the solid-antigen prepared with the addition of U1snRNA in addition to the recombinant RNP, compared to the conventional ELISA method (ELISA method using the antigen that only recombinant RNP was solid-phased). It is presumed that this is because the anti-U1RNP antibody for recognizing U1snRNA-RN complex in the dissociation specimen can be detected similar to the case measured by DID method.

Moreover, as for the specimens determined as positive (positive specimens) by both DID method and the conventional ELISA method, although there are some specimens whose reactivity is not enhanced even if the U1snRNA is added, with regard to this, it is considered because the relevant specimens include the anti-U1RNP antibody type that mainly recognizes only RNP. In the healthy persons' specimens, the change of the reactivity was not recognized by the addition of U1snRNA. from this fact, it is indicated that even if U1snRNA is added to the antigen solution, non-specific reaction does not exist. Therefore, even if a solution to which U1snRNA was added is used as the antigen solution, the healthy persons specimens are not determined as positive at all.

As described above, a measurement system that determines even the specimens determined as negative by the conventional ELISA method as positive is constructed by making the complex of U1snRNA and recombinant RNP as an antigen. If such a measurement system is used, the measurement having a high correlation with the conventional DID method is performed, and the anti-U1RNP in the specimen can be detected or measured with a high sensitivity.

### (Example 6) Comparison between ELISA method using only RNP and ELISA method using complex of RNP and U1snRNA

The comparison between the ELISA method using only RNP as a solid-phased antigen and the ELISA method using the complex of RNP and U1snRNA as a solid-phased antigen was carried out in the following manner.

First, after His-tagged RNP 70k (0.55 µg/mL), His-tagged RNPA (0.27 µg/mL), and His-tagged RNPC(0.18 µg/mL)obtained in Example 2 were incubated at 37°C for 5 minutes in PBS buffer, 100 µL each was dispensed to each well of the 96 wells micro-titer plate (Maxisorp Nunc), and left at 4°C over night, thereby absorbing the respective RNP on the plate. Subsequently, the antigen solution was removed from each well, and 200 µL of the PBS buffer was added to each well. After leaving for about 10 seconds, the PBS buffer was removed from each well. The unabsorbed RNP was removed by repeating this washing operation twice. Furthermore, 200 µL of the blocking buffer (1%BSA/PBS) was added to each well, and left at 4°C over night and the blocking was carried out. Subsequently, the blocking buffer was removed from each well, it was made a plate for ELISA measurement (RNP-ELISA plate) by sufficiently drying it.

On the other hand, similarly, after His-tagged RNP 70k (0.55 µg/mL), His-tagged RNPA (0.27 µg/mL), and His-tagged RNPC(0.18µg/mL)obtained in Example 2 and U1snRNA(10 µg/mL) obtained in Example 1 were incubated at 37°C for 5 minutes in PBS buffer,100 µL each was dispensed to each well of the 96 wells micro-titer plate (made by Maxisorp Nunc, Co., Ltd.), and left at 4°C over night, thereby absorbing the U1snRNA-RN protein complex on the plate. Subsequently, the antigen solution was removed from each well, and 200 µL of the PBS buffer was added to each well. After leaving for about 10 seconds, the PBS buffer was removed from each well. The unabsorbed U1snRNA RNP or the like was removed by repeating this washing operation twice. Furthermore, 200 µL of the blocking buffer (1%BSA/PBS) was added to each well, and left at 4°C over night and the blocking was carried out. Subsequently, the blocking buffer was removed from each well, it was made a plate for ELISA measurement (U1snRNA + RNP ELISA plate) by sufficiently drying it.

Using the plate for ELISA measurement (RNP-ELISA plate and U1snRNA + RNP ELISA plate) prepared as described above, the measurement on the 196 serum specimens of patients with collagen disease determined as positive and the healthy persons' 82 serum specimens determined as negative by the DID method of the above-described Reference Experiment Example 1 (DID method using ENA-1test (made by Medical Biological Institute, Co., Ltd.)) was carried out.

First, the primary reaction was carried out by adding 100 µL of the solution to which each specimen was diluted 100 times with the buffer for reaction to each well of the plate for ELISA measurement and by standing at 25°C for one hour. After the reaction solution was removed from each well, each well was sufficiently washed with PBS buffer containing 1% Tween-20. Subsequently, the secondary reaction was carried out by adding 100 µL of the labeled antibody solution containing anti-human IgG antibody which had been labeled with peroxidase (made by Medical Biological Institute, Co., Ltd.) to each well and by standing at 25°C for one hour. After the reaction solution was removed from each well, each well was sufficiently washed with PBS buffer containing 1% Tween-20. Subsequently, 100 µL of enzyme matrix liquid containing tetramethylbenzidine and hydrogen peroxide was added to each well, and the enzyme reaction was carried out at 25°C for 30 minutes. Subsequently, after 100 µL of 1N sulfuric acid was added to each well and the enzyme reaction was quenched, 450 nm of absorbance was measured by a spectrophotometer.

In Fig. 5, a graph representing of the measurement results are shown. Fig. 5(a) is a graph in which the measurement results in the case of using RNP-ELISA plate are plotted, while Fig 5(b) is a graph in which the measurement results in the case of using U1snRNA+RN ELISA plate. As shown in Fig. 5 (a), in the case where only RNP was used as a solid-phased antigen, the portion where the color development distribution in the specimen group determined as positive by the DID method (population on the left side) is overlapped with the color development distribution of the healthy persons' group determined as negative by the DID method (population on the right side) is generated (i.e., the specimen whose color development is in the similar degree with the specimen (healthy person's specimen) determined as negative by the DID method in the specimens determined as positive by DID method), it is understood that one portion of the specimens determined as positive by the DID method can not be determined as positive by the ELISA method using only RNP as a solid-phased antigen. In other words, the ELISA method using only RNP as a solid-phased antigen is shown to be short of the measurement sensitivity, compared to the DID method.

On the other hand, as shown in Fig. 5(b), in the measurement system using U1snRNA and RNP as solid-phased antigens, the color development distribution in the specimen group determined as positive by the DID method (population located on the left side) does not overlap with the color development distribution of the healthy persons' group determined as negative by the DID method (population located on the right side (i.e., the color development in the specimen determined as positive by the DID method is enhanced, compared to the color development in the healthy persons' specimen determined as negative by the DID method), it is indicated that the ELISA method using the relevant U1snRNA and RNP as solid-phased antigens has at least approximately the same degree of measurement sensitivity with that of the conventional DID method.

In Fig. 6, a graph in which the measurement results by the above-described two measurement systems are compiled and plotted is shown. In the relevant graphical representation, only the measurement results of the 196 serum specimens of patients with collagen disease determined as positive by the DID method are plotted. The abscissa axis represents color development (A450) by the ELISA methodusing only RNP as a solid-phased antigen, and the ordinate axis represents the color development (A450) by the ELISA method using U1snRNA and RNP as solid-phased antigens. From this graph, the positive correlation between both measurement systems is recognized.

As described above, it is recognized that the ELISA method using RNP and U1snRNA as solid-phased antigens has a correlation with the ELISA method using only RNP as a solid-phased antigen, and in addition to this, the ELISA method using RNP and U1snRNA as solid-phased antigens is a method for measurement having a high detection sensitivity, which can determine (detect) the specimen determined as positive by the conventional DID method, but determined as negative by the ELISA method using only RNP as a solid-phased antigen (dissociation specimen) as positive.

### INDUSTRIAL APPLICABILITY

As described above, in a method for measuring an anti-ENA antibody of the present invention, an anti-ENA antibody capable of recognizing an antigen in a state of existing in vivo can be detected with a high sensitivity. This means that a method for measurement having a high correlation with the DID method which has been conventionally defined as the standard method is provided. Moreover, since an antigen used for the method of measurement of the present invention can be easily prepared, the simplification of the operation is contemplated. Furthermore, since the measurement is performed using an antigen with a high purity, the measurement with a higher sensitivity is capable of being realized.

## Claims

1. A method for measuring an anti-ENA antibody which specifically recognizes an antigen containing a first RNA and a first intracellular non-histone soluble protein, comprising the steps of: forming a complex of a second RNA which is substantially the same as the first RNA and a first protein molecule which is substantially the same as the first intracellular non-histone soluble protein, and then reacting the complex and a specimen.

2. A method for measuring an anti-ENA antibody which specifically recognizes an antigen containing a first RNA, a first intracellular non-histone soluble protein and a second intracellular non-histone soluble protein and whose recognition site is located at anyone of the first RNA, the first intracellular non-histone soluble protein or a complex thereof, comprising the steps of: forming a complex of a second RNA which is substantially the same as the first RNA and a first protein molecule which is substantially the same as the first intracellular non-histone soluble protein, and then reacting the relevant complex and a specimen.

3. A method for measuring an anti-ENA antibody which specifically recognizes an antigen containing a first RNA and a first intracellular non-histone soluble protein, the method comprising the following steps a)-c):
a) forming a complex of a second RNA which is substantially the same as the first RNA and a first protein molecule which is substantially the same as the first intracellular non-histone soluble protein;
b) reacting a specimen with the complex; and
c) detecting a reaction product generated by the step b).

4. A method for measuring an anti-ENA antibody which specifically recognizes an antigen containing a first RNA, a first intracellular non-histone soluble protein and a second intracellular non-his tone soluble protein and whose recognition site is located at any one of the first RNA, the first intracellular non-histone soluble protein with a cell or a complex thereof, the method comprising the following steps a)-c):
a) forming a complex of a second RNA which is substantially the same as the first RNA and a first protein molecule which is substantially the same as the first intracellular non-histone soluble protein;
b) reacting a specimen with the complex; and
c) detecting a reaction product generated by the step b).

5. The method for measuring an anti-ENA antibody as claimed in any one of Claim 1 to 4, wherein the first intracellular non-histone soluble protein is a intracellular non-histone soluble protein selected from the group consisting of U1RNP, Sm, SS-A, SS-B, Jo-1 and PM-Scl.

6. The method for measuring an anti-ENA antibody as claimed in any one of Claim 1 to 5, wherein the second RNA has been prepared by a genetic engineering technique or chemical synthesis.

7. The method for measuring an anti-ENA antibody as claimed in any one of Claim 1 to 6, wherein the first protein molecule is a recombinant protein.

8. A complex used for measuring an anti-ENA antibody which specifically recognizes an antigen containing a first RNA and a first intracellular non-histone soluble protein, the complex comprising a second RNA which is substantially the same as the first RNA and a first protein molecule which is substantially the same as the first intracellular non-histone soluble protein.

9. A complex used for measuring an anti-ENA antibody which specifically recognizes an antigen containing a first RNA, a first intracellular non-histone soluble protein and a second intracellular non-histone soluble protein and whose recognition site is located at any one of the RNA, the first intracellular non-histone soluble protein or a complex thereof, comprising:
a second RNA which is substantially the same as the first RNA and a first protein molecule which is substantially the same as the first intracellular non-histone soluble protein.

10. The complex as claimed in Claim 8 or Claim 9, wherein the first intracellular non-histone soluble protein is a intracellular non-histone soluble protein selected from the group consisting of U1RNP, Sm, SS-A, SS-R, Jo-1 and PM-Scl.

11. The complex as claimed in any one of Claim 8 to 10, wherein the second RNA has been prepared by a genetic engineering technique or chemical synthesis.

12. The complex as claimed in any one of Claim 8 to 11, wherein the first protein molecule is a recombinant protein.

13. A solid-phased antigen for measuring an anti-ENA body binding the complex claimed in any one of Claim 8 to 12 to an insoluble support.

14. A kit for measuring an anti-ENA antibody which specifically recognizes an antigen containing a first RNA and a first intracellular non-histone soluble protein, comprising a solid-phased antigen which has solid-phased a complex of a second RNA which is substantially the same as the first RNA and a first protein molecule which is substantially the same as the first intracellular non-histone soluble protein on an insoluble support; an anti-human immune globulin antibody; and the standard specimen containing an anti-ENA antibody.

15. A kit for measuring an anti-ENA antibody which specifically recognizes an antigen containing a first RNA, a first intracellular non-histone soluble protein and a second intracellular non-histone soluble protein and whose recognition site is located at any one of the RNA, the first intracellular non-histone soluble protein or a complex thereof, comprising:
a solid-phased antigen which has solid-phased a complex of a second RNA which is substantially the same as the first RNA and a first protein molecule which is substantially the same as the first intracellular non-histone soluble protein on an insoluble support; an anti-human immune globulin antibody; and the standard specimen containing an anti-ENA antibody.

16. The kit for measuring an anti-ENA antibody as claimed in Claim 14 or Claim 15, wherein the first intracellular non-histone soluble protein is a intracellular non-histone soluble protein selected from the group consisted of U1RNP, Sm, SS-A, SS-R, Jo-1 and PM-Scl.

17. The kit for measuring an anti-ENA antibody as claimed in any one of Claim 14 to 16, wherein the second RNA has been prepared by a genetic engineering technique or chemical synthesis.

18. The kit for measuring an anti-ENA antibody as claimed in any one of Claim 14 -Claim 17, wherein the first protein molecule is a recombinant protein.

19. Amethod formeasuring an anti-U1RNP which specifically recognizes an U1RNP consisted of an U1snRNA and an RNP, comprising:
the step of forming a complex of an RNA which is substantially the same as the U1snRNA and a protein molecule which is substantially the same as the RNP, and then reacting the relevant complex and a specimen.

20. A method for measuring an anti-U1RNP antibody which specifically recognizes an U1RNP consisted of an U1snRNA and an RNP, comprising the following steps A)-C):
A) forming a complex of an RNA which is substantially the same as the U1snRNA and a protein molecule which is substantially the same as the RNP,
B) reacting a specimen with the complex, and
C) detecting a reaction product generated by the step B).

21. The method for measuring an anti-U1RNP antibody as claimed in Claim 19 or 20, wherein the RNA has been prepared by a genetic engineering technique or chemical synthesis.

22. The method for measuring an anti-U1RNP antibody as claimed in any one of Claim 19 to 21, wherein the protein molecule is a recombinant protein.

23. A complex comprising an RNA which is substantially the same as an U1snRNA and a protein molecule which is substantially the same as an RNP.

24. The complex as claimed in Claim 23, wherein the RNA has been prepared by a genetic engineering technique or chemical synthesis.

25. The complex as claimed in Claim 23 or Claim 24, wherein the first protein molecule is a recombinant protein.

26. A solid-phased antigen for measuring an anti-U1RNP antibody which binds the complex claimed in any one of Claim 23 to 25 on an insoluble support.

27. A kit for measuring an anti-U1RNP antibody, the kit comprising:
a solid-phased antigen that has solid-phased a complex of an RNA which is substantially the same as an U1snRNA and a protein molecule which is substantially the same as an RNP on an insoluble support, an anti-human immune globulin antibody and the standard specimen containing an anti-U1RNP antibody.

28. The kit for measuring an anti-U1RNP antibody as claimed in Claim 27, wherein the RNA has been prepared by a genetic engineering technique or chemical synthesis.

29. The kit for measuring an anti-U1RNP antibody as claimed in Claim 27 or Claim 28, wherein the protein molecule is a recombinant protein.
